Europäisches Patentamt

European Patent Office

Office européen des brevets

(11) Veröffentlichungsnummer: **0 184 726**
**A2**

(12) # EUROPÄISCHE PATENTANMELDUNG

(21) Anmeldenummer: **85115133.2**

(22) Anmeldetag: **29.11.85**

(51) Int. Cl.⁴: **C 07 D 233/61**, C 07 D 249/08, A 01 N 43/50, A 01 N 43/653 // C07D233/60, C07C49/175

(30) Priorität: **11.12.84 DE 3445089**

(43) Veröffentlichungstag der Anmeldung: **18.06.86 Patentblatt 86/25**

(84) Benannte Vertragsstaaten: **AT BE CH DE FR GB IT LI NL**

(71) Anmelder: **BAYER AG, Konzernverwaltung RP Patentabteilung, D-5090 Leverkusen 1 Bayerwerk (DE)**


(72) Erfinder: **Jäger, Gerhard, Dr., Paul-Klee-Strasse 28j, D-5090 Leverkusen 1 (DE)**
Erfinder: **Jautelat, Manfred, Dr., Müllersbaum 28, D-5093 Burscheid 1 (DE)**
Erfinder: **Elbe, Hans-Ludwig, Dr., Dasnöckel 59, D-5600 Wuppertal 11 (DE)**
Erfinder: **Reinecke, Paul, Dr., Steinstrasse 8, D-5090 Leverkusen 3 (DE)**


(54) **Azolyl-alkanonoxim-Derivate.**


(57) Die Erfindung betrifft neue Azolyl-alkanonoxim-Derivate, mehrere Verfahren zu deren Herstellung und deren Verwendung als Fungizide.

Die neuen Verbindungen der Formel

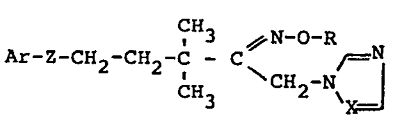

in welcher

Ar, Z, R und X die in der Beschreibung angegebene Bedeutung besitzen,

werden erhalten, wenn man die entsprechenden Azolyl-keto-Verbindungen mit Hydroxylamin oder Hydroxylamin-Derivaten umsetzt. Daneben gibt es noch andere Verfahrensvarianten.

Die neuen Verbindungen lassen sich als Pflanzenschutzmittel einsetzen. Sie können insbesondere zur Bekämpfung von Pflanzenkrankheiten in Getreide- und Reiskulturen Verwendung finden.


EP 0 184 726 A2

BAYER AKTIENGESELLSCHAFT 5090 Leverkusen, Bayerwerk

Konzernverwaltung RP
Patentabteilung

Slr/Ke-c

I

## Azolyl-alkanonoxim-Derivate

Die vorliegende Erfindung betirfft neue Azolyl-alkanonoxim-Derivate, mehrere Verfahren zu deren Herstellung und deren Verwendung als Fungizide.

Es ist bereits bekannt geworden, daß man Azolylmethyloxime als Pflanzenschutzmittel mit funiqzider Wirkunq verwenden kann (vgl. EP-A 0 094 572).
Die Wirkung dieser Verbindungen ist jedoch, insbesondere bei niedrigen Anwendungskonzentrationen, nicht immer voll befriedigend.

Es wurden nun die neuen Azolyl-alkanonoxim-Derivate der allgemeinen Formel (I)

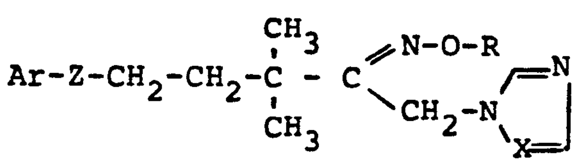

(I)

in welcher

X für Stickstoff und die CH-Gruppe steht,

Z für Sauerstoff, Schwefel, die -SO- und die $-SO_2$-Gruppe steht,

R für Wasserstoff, Alkyl, Alkenyl, Alkinyl, und gegebenenfalls substituiertes Benzyl steht, und

Ar für gegebenenfalls substituiertes Aryl steht,

und deren Säureadditions-Salze und Metallsalz-Addukte gefunden.

Man erhält die Azolyl-alkanonoxim-Derivate der Formel (I)

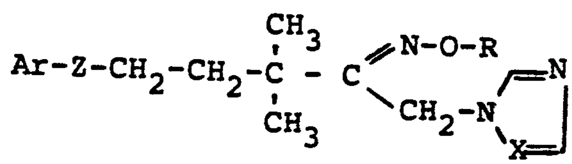

(I)

in welcher

X für Stickstoff und die CH-Gruppe steht,

Z für Sauerstoff, Schwefel, die -SO- und die $-SO_2$-Gruppe steht,

R für Wasserstoff, Alkyl, Alkenyl, Alkinyl, und gegebenenfalls substituiertes Benzyl steht, und

Ar für gegebenenfalls substituiertes Aryl steht,
sowie deren Säureadditions-Salze und Metallsalz-Komplexe,

wenn man

a) Azolyl-keto-Verbindungen der Formel (II)

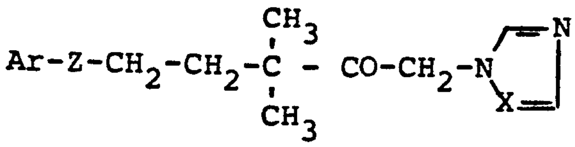

(II)

in welcher

Ar, Z und X die oben angegebene Bedeutung besitzen,

mit Hydroxylamin in Gegenwart eines Verdünnungsmittels umsetzt und gegebenenfalls noch die erhaltenen Verbindungen der Formel (Ia)

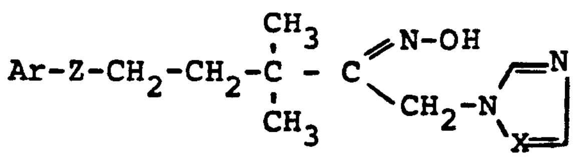

(Ia)

(das sind solche Verbindungen der Formel (I), bei denen R für Wasserstoff steht)

mit einer Halogen-Verbindung der Formel (III)

R-Hal (III)

in welcher

Hal für Halogen, wie Chlor oder Brom, steht, und

R die obengenannten Bedeutungen besitzt,

in Gegenwart eines Verdünnungsmittels und eines Säurebindemittels zur Reaktion bringt,

oder

b) die Azolyl-keto-Verbindungen der Formel (II) mit einem Hydroxylamin-Derivat der Formel (IV)

$$R\text{-}O\text{-}NH_2 \qquad (IV)$$

in welcher

R die obengenannte Bedeutung besitzt,

in Gegenwart eines Verdünnungsmittels umsetzt,

und gegebenenfalls noch an die erhaltenen Verbindungen der Formel (I) anschließend eine Säure oder ein Metallsalz addiert.

Die neuen Azolyl-alkanonoxim-Derivate der Formel (I) weisen starke fungizide Eigenschaften auf. Überraschenderweise zeigen dabei die Verbindungen der Formel (I) eine erheblich höhere fungizide Wirkung als die aus dem Stande der Technik bekannten Azolylmethyloxime.

Von den erfindungsgemäßen Azolylalkanonoxim-Derivaten der Formel (I) sind bevorzugt diejenigen Verbindungen in denen

R für Wasserstoff, für geradkettiges oder verzweigtes Alkyl, Alkenyl und Alkinyl mit jeweils bis zu 4 Kohlenstoffatomen steht, ferner für Benzyl steht, welches gegebenenfalls im Phenylteil durch Halogen und/oder Alkyl mit 1 bis 3 Kohlenstoffatomen substituiert sein kann,

Ar für Phenyl steht, welches gegebenenfalls durch Halogen, Nitro, durch gegebenenfalls halogensubstituiertes Phenyl und/oder durch Halogenalkyl, Halogenalkoxy oder Halogenalkylthio substituiert sein kann, wobei die drei letztgenannten Substituenten 1 bis 2 Kohlenstoff- und 1 bis 5 Halogenatome enthalten können, und

X und Z die in der Erfindungsdefinition genannte Bedeutung besitzen.

Besonders bevorzugt sind diejenigen Azolyl-alkanonoxim-Derivate der allgemeinen Formel (I), in denen

R für Wasserstoff, Methyl, Ethyl, Isopropyl und tert.-Butyl, ferner für Benzyl steht, welches im Phenylteil ein- bis dreifach durch Fluor, Chlor, Methyl, Ethyl und/oder Isopropyl substituiert sein kann,

Ar für Phenyl steht, welches gegebenenfalls einfach durch Phenyl, Mono- oder Dichlor-phenyl oder Mono- oder Difluor-phenyl, ferner ein- bis dreifach durch Fluor, Chlor, Nitro, Trifluormethyl, Trifluormethoxy und/oder Trifluormethylthio substituiert sein kann, und

X und Z die in der Erfindungsdefinition genannte Bedeutung besitzen.

Im einzelnen seien folgende Verbindungen der allgemeinen Formel (I) genannt:

| Ar | R | X | Z |
|---|---|---|---|
| 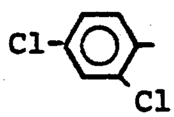 | H | CH | O |
| " | $CH_3$ | CH | O |
| " | Allyl | CH | O |
| " | Propargyl | CH | O |
| " | H | N | O |
| " | $CH_3$ | N | O |
| " | $i-C_3H_7$ | N | O |
| " | Allyl | N | O |
| " | 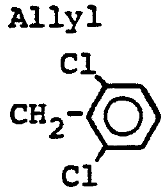 | N | O |

| Ar | R | X | Z |
|---|---|---|---|
| 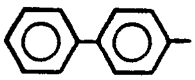 | H | CH | O |
| " | H | N | O |
| " | $C_2H_5$ | N | O |
| " | $i-C_3H_7$ | N | O |
| " | 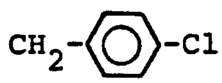 | N | O |
| 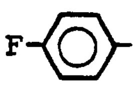 | H | CH | O |
| " | H | N | O |
| " | $CH_3$ | N | O |
| " | $C_2H_5$ | N | O |
| " | Propargyl | N | O |
| 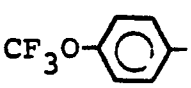 | H | CH | O |
| " | H | N | O |
| 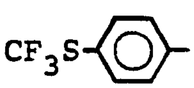 | H | CH | O |
| " | H | N | O |
| " | $C_2H_5$ | N | O |
| " | Allyl | N | O |

| Ar | R | X | Z |
|---|---|---|---|
| 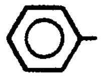 | H | CH | S |
| " | H | N | S |
| " | H | CH | SO |
| " | H | N | SO |
| " | H | CH | $SO_2$ |
| " | H | N | $SO_2$ |

Verwendet man zur Herstellung der erfindungsgemäßen Stoffe der Formel (Ia) nach Verfahrensvariante a) beispielsweise 5-(4-Chlorphenoxy)-3,3-dimethyl-1-(1,2,4-triazol-1-yl)-pentan-2-on und Hydroxylamin als Ausgangsstoffe, so kann der Reaktionsablauf durch das folgende Formelschema wiedergegeben werden:

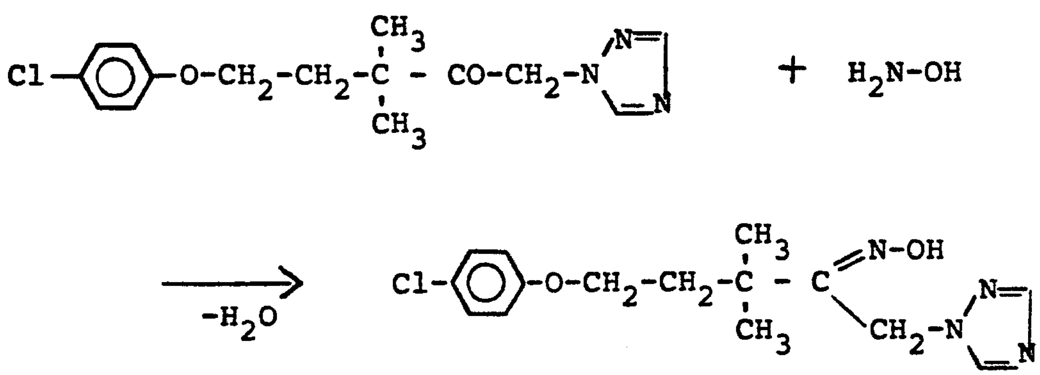

Die nachfolgende Alkinylierung mit beispielsweise Propargylbromid zu einer Verbindung der Formel (I) läßt sich dann wie folgt wiedergeben:

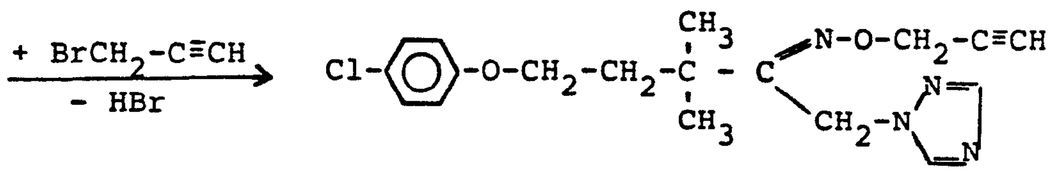


Verwendet man zur Herstellung der Stoffe der Formel (I) nach Verfahrensvariante b) beispielsweise 5-(4-Phenyl-phenoxy)-3,3-dimethyl-1-(1,2,4-triazol-1-yl)-pentan-2-on und O-Methyl-hydroxylamin als Ausgangsstoffe, so kann der Reaktionsablauf durch das folgende Formelschema wiedergegeben werden:

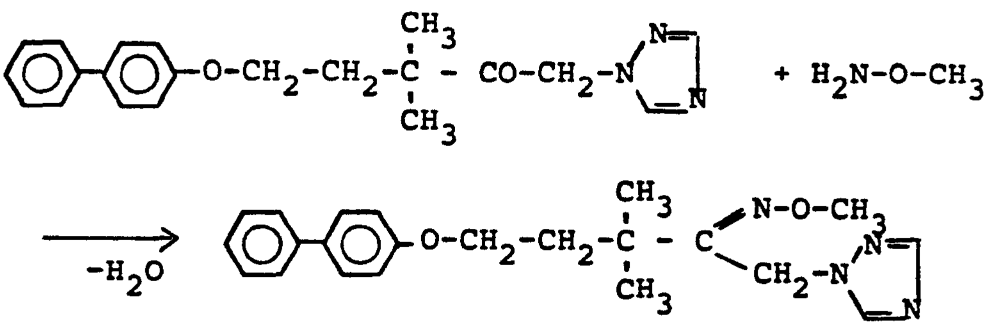


Die zur Herstellung der erfindungsgemäßen Azolyl-alkanoxim-Derivate als Ausgangsstoffe benötigten Azolylketo-Verbindungen sind durch die Formel (II) allgemein definiert. In der Formel (II) steht Ar vorzugsweise für die weiter oben angegebenen bevorzugten Bedeutungen.

Die Azolyl-keto-Verbindungen der Formel (II) sind bereits in der Literatur beschrieben (vgl. z.B. EP-A 0 054 865 , EP-A 0 096 801 und DE-A 3 335 767 . Sie werden nach im Prinzip bekannten Verfahren hergestellt, indem man die entsprechenden Halogen-keto-Verbindungen mit dem entsprechenden Azol, wie Imidazol oder 1,2,4-Triazol, in Gegenwart eines inerten organischen Lösungsmittels, wie beispielsweise Aceton oder Acetonitril, und in Gegenwart eines Säurebindemittels, wie beispielsweise Kaliumcarbonat oder Triethylamin, bei Temperaturen zwischen 20 und 150°C umsetzt.

Das weiterhin als Ausgangsstoff für die Verfahrensvariante a) benötigte Hydroxylamin und die für die Alkylierung bzw. Alkenylierung bzw. Alkinylierung bzw. Benzylierung benötigten Halogen-Verbindungen der Formel (III) sind allgemein bekannte Stoffe.

Die sodann als Ausgangsstoffe für die Verfahrensvariante b) benötigten Hydroxylamin-Derivate der Formel (IV) sind ebenfalls bekannte Verbindungen (vgl. z.B. die EP-A 0 015 456).

In den Formeln (III) und (IV) besitzt der Substituent R vorzugsweise die weiter oben angegebenen bevorzugten Bedeutungen.

Als Verdünnungsmittel kommen alle inerten organischen Lösungsmittel für das erfindungsgemäße Verfahren gemäß Verfahrensvarianten a) und b) infrage.

Zu nennen sind hier Kohlenwasserstoffe wie Benzin und Toluol, Ether, wie Diethylether, Dioxan und Tetrahydrofuran, Nitrile, wie insbesondere Acetonitril, Alkohole, wie Methanol und Ethanol. Insbesondere eignet sich Dimethylformamid für die erfindungsgemäße Umsetzung.

Setzt man gemäß Verfahrensvariante a) Hydroxylamin bzw. gemäß Verfahrensvariante b) Hydroxylamin-Derivate der Formel (IV) in Form von Salzen, z.B. als Hydrochloride, ein, so sind zur Durchführung der Reaktion Säurebinder erforderlich. Hierzu können übliche Säurebindemittel verwendet werden, wie z.B. Alkalihydroxide, Alkalicarbonate oder Alkalisalze organischer Säuren, wie z.B. Natriumacetat. Arbeitet man gemäß Verfahrensvariante a) und setzt primär erhaltene Oxime der Formel (Ia) mit einer Halogen-Verbindung der Formel (III) um, so sind ebenfalls Säurebinder zu verwenden. Zu nennen sind hier Alkali-alkoholate, wie z.B. Natriummethylat oder Kalium-tert.-butylat.

Die Reaktionstemperaturen können in einem größeren Bereich variiert werden. Setzt man gemäß Verfahrensvariante a) Azolyl-keto-Verbindungen der Formel (II) mit Hydroxylamin bzw. gemäß Verfahrensvariante b) mit Hydroxylamin-Derivaten der Formel (IV) um, so arbeitet man im allgemeinen zwischen etwa 0 und +80°C, vorzugsweise zwischen +10 und 60°C. Arbeitet man gemäß Verfahrensvariante a) und setzt die primär erhaltenen Oxime der Formel (Ia) mit einer Halogen-Verbindung der Formel (III) um, so arbeitet man im allgemeinen zwischen +20 und 120°C, vorzugsweise zwischen +30 und 80°C.

Bei der Durchführung des erfindungsgemäßen Verfahrens gemäß Verfahrensvariante a) setzt man auf 1 Mol Azolyl-keto-Verbindung der Formel (II) etwa 1,5 bis 4 Mol Hydroxylamin ein. In entsprechender Weise werden gemäß Verfahrensvariante b) auf 1 Mol der Verbindung der Formel (II) etwa 1,5 bis 4 Mol Hydroxylamin-Derivate der Formel (IV) eingesetzt. Arbeitet man gemäß Verfahrensvariante a) und setzt die primär erhaltenen Oxime der Formel (Ia) mit einer Halogen-Verbindung der Formel (III) um, so setzt man auf 1 Mol Oxim etwa 1,2 bis 3 Mol der Halogenverbindung ein. Die Aufarbeitung erfolgt in allgemein üblicher Weise nach bekannten Methoden (vgl. auch die Herstellungsbeispiele).

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Botrytis-Arten, wie beispielsweise Botrytis cinerea;
Plasmopara-Arten, wie beispielsweise Plasmopara viticola;

Uromyces-Arten, wie beispielsweise Uromyces appendiculatus;
Sphaerotheca-Arten, wie beispielsweise Sphaerotheca fuliginea;
Venturia-Arten, wie beispielsweise Venturia inaequalis;
Podosphaera-Arten, wie beispielsweise Podosphaera leucotricha;
Phytophthora-Arten, wie beispielsweise Phytophthora infestans;
Erysiphe-Arten, wie beispielsweise Erysiphe graminis;
Puccinia-Arten, wie beispielsweise Puccinia recondita;
Drechslera-Arten, wie beispielsweise Drechslera graminea (synonym mit Helminthosporium);
Fusarium-Arten, wie beispielsweise Fusarium culmorum;
Ustilago-Arten, wie beispielsweise Ustilago nuda oder Ustilago avenae;
Septoria-Arten, wie beispielsweise Septoria nodorum;
Tilletia-Arten, wie beispielsweise Tilletia caries;
Xanthomonas-Arten, wie beispielsweise Xanthomonas oryzae;
Pseudomonas-Arten, wie beispielsweise Pseudomonas lachrymans;
Pyricularia-Arten, wie beispielsweise Pyricularia oryzae;
Pellicularia-Arten, wie beispielsweise Pellicularia sasakii,
Pyrenophora-Arten, wie beispielsweise Pyrenophora teres (Konidienform: Drechslera, Synonym: Helminthosporium),
Leptosphaeria-Arten, wie beispielsweise Leptosphaeria nodorum;
Cochliobolus-Arten, wie beispielsweise Cochliobolus sativus, (Konidienform: Drechslera, Synonym: Helminthosporium);
Cercospora-Arten, wie beispielsweise Cercospora canescens.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Verbindungen sind besonders gut wirksam gegen Krankheiten in Getreidekulturen, wie beispielsweise gegen den Erreger des Getreidemehltaus (Erysiphe graminis), gegen Septoria-Arten, Cochliobolus sativus und Pyrenophora teres, weiterhin gegen Schorf- und Rostpilze sowie gegen Botrytis-Arten und gegen den Reispflanzen schädigenden Pilz Pyricularia oryzae.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase,wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additve können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen,

Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

0184726

Herstellungsbeispiele

Beispiel 1

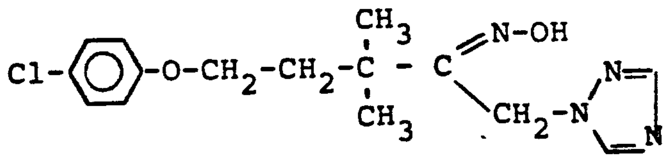

Zu einer Lösung von 117 g (0,38 Mol) 5-(4-Chlorphenoxy)-3,3-dimethyl-1-(1,2,4-triazol-1-yl)-2-pentanon in 2000 ml N,N-Dimethylformamid werden bei Raumtemperatur 72,3 g (1,04 Mol) Hydroxylaminhydrochlorid und 170 g (1,25 Mol) Natriumacetat zugegeben. Nach 48 Stunden wird das Reaktionsgemisch filtriert, das Filtrat im Vakuum eingeengt und der Rückstand mit 1000 ml Wasser versetzt. Man extrahiert mit 500 ml Dichlormethan, trocknet die organische Phase über Natriumsulfat und destilliert das Lösungsmittel unter vermindertem Druck ab. Es werden so 86 g (70,1 % der Theorie) an 5-(4-Chlorphenoxy)-3,3-dimethyl-1-(1,2,4-triazol-1-yl)-2-pentanonoxim als farblose Kristalle vom Schmelzpunkt 104 bis 105°C erhalten.

Herstellung der Vorprodukte

Vorprodukt 1:

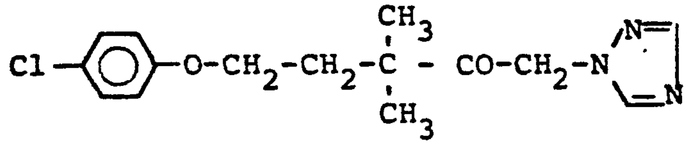

Zu einem Gemisch aus 27,6 g (0,2 Mol) Kaliumcarbonat und 27,6 g (0,4 Mol) 1,2,4-Triazol in 400 ml siedendem Aceton wird eine Lösung von 55 g (0,2 Mol) 1-Chlor-5-(4-chlorphenoxy)-3,3-dimethyl-pentan-2-on in 50 ml Aceton zugetropft. Man hält das Gemisch noch 3 Stunden am Sieden, kühlt auf 20°C ab, filtriert und engt das Filtrat im Vakuum ein. Der ölige Rückstand wird in 250 ml Essigester aufgenommen und dreimal mit je 100 ml Wasser gewaschen. Nach dem Trocknen der organischen Phase über wasserfreiem Natriumsulfat wird im Vakuum eingedampft. Das verbleibende Rohprodukt (61 g) wird in 250 ml Aceton gelöst und mit einer Lösung von 57,6 g Naphthalin-1,5-disulfonsäure in 250 ml Aceton versetzt. Das auskristallisierte Salz (45,4 g; Schmelzpunkt 188 bis 190°C) wird in einem Gemisch aus je 150 ml Dichlormethan und Wasser suspendiert und durch Zugabe von 10 %iger Natriumcarbonat-Lösung alkalisch gestellt. Man trennt die organische Phase ab, extrahiert die wäßrige Phase noch einmal mit 100 ml Dichlormethan und engt die vereinigten organischen Phasen im Vakuum ein. Man erhält 30,4 g (49,4 % der Theorie) 5-(4-Chlorphenoxy)-3,3-dimethyl-1-(1,2,4-triazol-1-yl)-pentan-2-on als schwach gelbliche Flüssigkeit vom Brechnungsindex $n_D^{20}$ = 1,5457.

Vorprodukt 2:

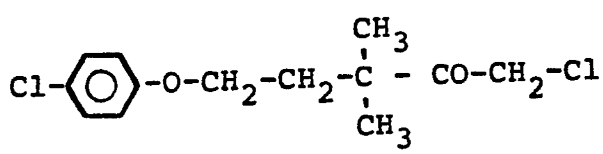

580 g (1,5 Mol) 1-Chlor-3,3-dimethyl-1,5-di-(4-chlorphenoxy)-1-pentan werden in 1000 ml Ethanol und 300 ml konzentrierter Salzsäure 4 Stunden unter Rückfluß erhitzt. Man zieht das Ethanol im Vakuum ab, verdünnt mit Dichlormethan und schüttelt mit Wasser, dann dreimal mit verdünnter Natronlauge aus. Die getrocknete Lösung wird im Vakuum vom Lösungsmittel befreit. Man erhält 363 g (88 % der Theorie) 1-Chlor-5-(4-chlorphenoxy)-3,3-dimethyl-pentan-2-on als Öl.

NMR ($CDCl_3$): = 1,25 (s, 6H), 2,1 (t, 2H, J=6 Hz), 3,9 (t, 2H, J= 6Hz), 4,45 (s, 2H), 6,7-7,3 (m, 4H)

Beispiel 2

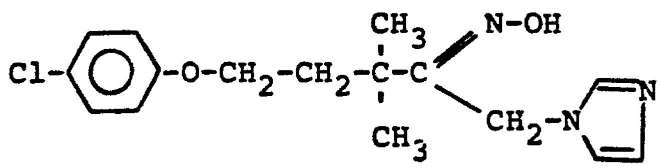

Eine Lösung von 298 g (0,97 Mol) 5-(4-Chlorphenoxy)-3,3-dimethyl-1-( imidazol-1-yl)-2-pentanon in 600 ml N,N-Dimethylformamid wird mit 159,4 g (2,3 Mol) Hydroxylaminhydrochlorid und 375,3 g (2,76 Mol) Natriumacetat versetzt. Nach 48-stündigem Rühren bei Raumtemperatur wird vom ausgeschiedenen Natriumchlorid abfiltriert, das Filtrat im Vakuum eingeengt und der Rückstand in 750 ml Dichlormethan aufgenommen. Die Lösung wird dreimal mit je 500 ml Wasser gewaschen, dann über Natriumsulfat getrocknet und im Vakuum eingedampft. Durch Verreiben des verbleibenden flüssigen Rückstands mit Diethylether erhält man 140 g (44,9 % der Theorie) 5-(4-Chlorphenoxy)-3,3-dimethyl-1-(imidazol-1-yl)-2-pentanonoxim als farblose Kristalle vom Schmelzpunkt 120 bis 121°C.

Beispiel 3

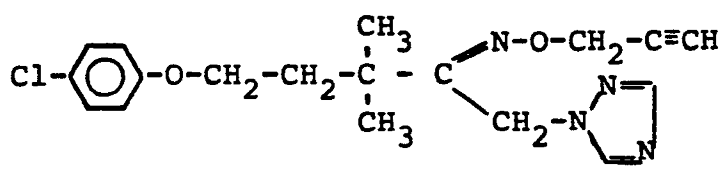

Zu einer auf 60°C erwärmten Lösung von 19,36 g (0,06 Mol) 5-(4-Chlorphenoxy)-3,3-dimethyl-1-(1,2,4-triazol-1-yl)-2-pentanonoxim und 6,72 g (0,06 Mol) Kaliumtertiärbutylat in 60 ml N,N-Dimethylformamid werden unter Rühren 14,3 g (0,12 Mol) Propargylbromid zugetropft. Man läßt noch 16 Stunden bei 60°C rühren, filtriert und engt das Filtrat im Vakuum ein. Der Rückstand wird in 200 ml Dichlormethan aufgenommen, die Lösung dreimal mit je 300 ml Wasser gewaschen, über Natriumsulfat getrocknet und im Vakuum eingedampft. Durch Chromatographie des flüssigen Rückstandes über Kieselgel mit Trichlormethan als Elutionsmittel werden 10,1 g (42 % der Theorie) 5-(4-Chlorphenoxy)-3,3-dimethyl-2-propargyloximino-1-(1,2,4-triazol-1-yl)-pentan als gelbliches Öl mit einem Brechungsindex $n_D^{23}$ = 1,5402 erhalten.

In entsprechender Weise werden die folgenden Verbindungen der allgemeinen Formel (I)

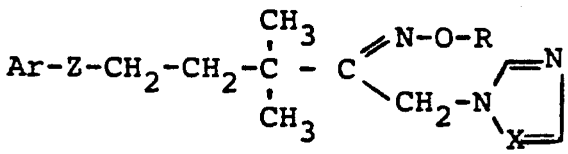

(I)

erhalten:

| Beispiel Nr. | Ar | Z | R | X | Physik. Daten Fp. (°C) oder $n_D^{23}$ |
|---|---|---|---|---|---|
| 4 | 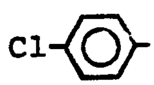 | O | $C_2H_5$ | N | 1,5338 |
| 5 | 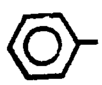 | O | H | CH | 128 - 129 |
| 6 | 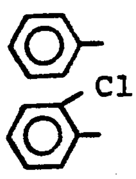 | O | H | N | 77 - 78 |
| 7 | | O | H | CH | 152 - 153 |

Verwendungsbeispiele

In den nachfolgenden Verwendungsbeispielen wird als Vergleichsubstanz verwendet:

(A) 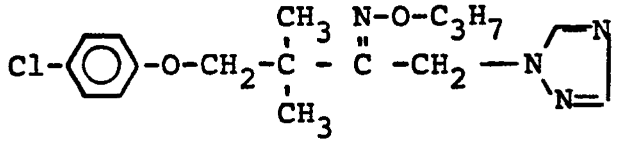

Beispiel A

Cochliobolus sativus-Test (Gerste) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Cochliobolus sativus besprüht. Die Pflanzen verbleiben 48 Stunden bei 20°C und 100 % rel. Luftfeuchtigkeit in einer Inkubationskabine.

Die Pflanzen werden in einem Gewächshaus bei einer Temperatur von 20°C und einer relativen Luftfeuchtigkeit von ca. 80 % aufgestellt.

7 Tage nach der Inokulation erfolgt die Auswertung.

Eine deutliche Überlegenheit in der Wirksamkeit gegenüber den Stand der Technik zeigen bei diesem Test z.B. die Verbindungen gemäß folgender Herstellungsbeispiele: 1, 4, 2.

Patentansprüche

1. Azolyl-alkanonoxim-Derivate der allgemeinen Formel (I)

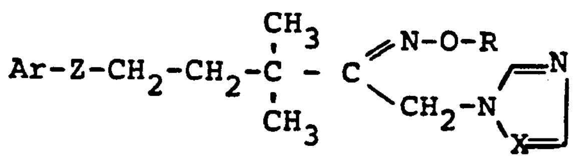

(I)

in welcher

X für Stickstoff und die CH-Gruppe steht,

Z für Sauerstoff, Schwefel, die -SO- und die $-SO_2$-Gruppe steht,

R für Wasserstoff, Alkyl, Alkenyl, Alkinyl, und gegebenenfalls substituiertes Benzyl steht, und

Ar für gegebenenfalls substituiertes Aryl steht,

und deren Säureadditions-Salze und Metallsalz-Addukte.

2. Verbindungen der allgemeinen Formel (I) in Anspruch 1, wobei

R für Wasserstoff, für geradkettiges oder verzweigtes Alkyl, Alkenyl und Alkinyl mit jeweils bis zu 4 Kohlenstoffatomen steht, ferner für

Benzyl steht, welches gegebenenfalls im Phenylteil durch Halogen und/oder Alkyl mit 1 bis 3 Kohlenstoffatomen substituiert sein kann,

Ar für Phenyl steht, welches gegebenenfalls durch Halogen, Nitro, durch gegebenenfalls halogensubstituiertes Phenyl und/oder durch Halogenalkyl, Halogenalkoxy oder Halogenalkylthio substituiert sein kann, wobei die drei letztgenannten Substituenten 1 bis 2 Kohlenstoff- und 1 bis 5 Halogenatome enthalten können, und

X und Z die in der Erfindungsdefinition genannte Bedeutung besitzen.

3. Verbindungen der allgemeinen Formel (I) in Anspruch 1, wobei

R für Wasserstoff, Methyl, Ethyl, Isopropyl und tert.-Butyl, ferner für Benzyl steht, welches im Phenylteil ein- bis dreifach durch Fluor, Chlor, Methyl, Ethyl und/oder Isopropyl substituiert sein kann,

Ar für Phenyl steht, welches gegebenenfalls einfach durch Phenyl, Mono- oder Dichlor-phenyl oder Mono- oder Difluor-phenyl, ferner ein- bis dreifach durch Fluor, Chlor, Nitro, Trifluormethyl, Trifluormethoxy und/oder Trifluormethylthio substituiert sein kann, und

X und Z die in der Erfindungsdefinition genannte Bedeutung besitzen.

4. Verfahren zur Herstellung von Azolyl-alkanonoxim-Derivaten der Formel (I)

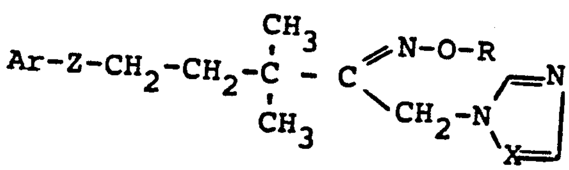

in welcher

X für Stickstoff und die CH-Gruppe steht,

Z für Sauerstoff, Schwefel, die -SO- und die $-SO_2$-Gruppe steht,

R für Wasserstoff, Alkyl, Alkenyl, Alkinyl und gegebenenfalls substituiertes Benzyl steht, und

Ar für gegebenenfalls substituiertes Aryl steht,

sowie von deren Säureadditions-Salzen und Metallsalz-Komplexen,

dadurch gekennzeichnet, daß man

a) Azolyl-keto-Verbindungen der Formel (II)

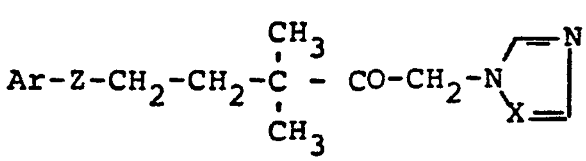

(II)

in welcher

Ar, Z und X die oben angegebene Bedeutung besitzen,

mit Hydroxylamin in Gegenwart eines Verdünnungsmittels umsetzt und gegebenenfalls noch die erhaltenen Verbindungen der Formel (Ia)

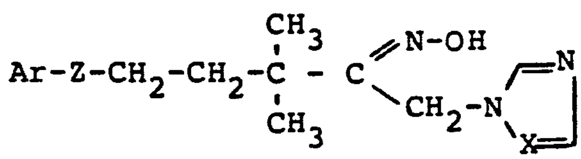

(Ia)

mit einer Halogen-Verbindung der Formel (III)

R-Hal (III)

in welcher

Hal für Halogen steht
und
R die obengenannten Bedeutungen besitzt,

in Gegenwart eines Verdünnungsmittels und eines Säurebindemittels zur Reaktion bringt,

oder

b) die Azolyl-keto-Verbindungen der Formel (II) mit einem Hydroxylamin-Derivat der Formel (IV)

$$R\text{-}O\text{-}NH_2 \qquad (IV)$$

in welcher

R die obengenannte Bedeutung besitzt,

in Gegenwart eines Verdünnungsmittels umsetzt,

und gegebenenfalls noch an die erhaltenen Verbindungen der Formel (I) anschließend eine Säure oder ein Metallsalz addiert.

5. Fungizide Mittel, gekennzeichnet durch einen Gehalt an mindestens einem Azolyl-alkanonoxim-Derivat der Formel (I) oder eines Säureadditions-Salzes bzw. Metallsalz-Komplexes eines Azolyl-alkanonoxim-Derivates der Formel (I).

6. Verfahren zur Bekämpfung von Pilzen, dadurch gekennzeichnet, daß man Azolyl-alkanonoxim-Derivate der Formel (I) bzw. deren Säureadditions-Salze oder Metallsalz-Komplexe auf Pilze und ihren Lebensraum einwirken läßt.

7. Verwendung von Azolyl-alkanonoxim-Derivaten der Formel (I) bzw. von deren Säureadditions-Salzen oder Metallsalz-Komplexen als Pflanzenschutzmittel.

8. Verwendung von Azolyl-alkanonoxim-Derivaten der Formel (I) bzw. von deren Säureadditions-Salzen oder Metallsalz-Komplexen zur Bekämpfung von Pilzen.

9. Verfahren zur Herstellung von fungiziden Mitteln, dadurch gekennzeichnet, daß man Azolyl-alkanonoxim-Derivate der Formel (I) bzw. deren Säureadditions-Salze oder Metallsalz-Komplexe mit Streckmitteln und/ oder oberflächenaktiven Mitteln vermischt.